# EUROPÄISCHE PATENTSCHRIFT

(11) **EP 1 793 775 B1**
(45) Veröffentlichungstag und Bekanntmachung des Hinweises auf die Patenterteilung: **27.04.2016**
(21) Anmeldenummer: 05772177.1
(22) Anmeldetag: 11.08.2005
(51) Int. Cl.: A61F 7/00, A61F 7/02, A61F 7/10

(54) **AUFLAGE ZUM KÜHLEN VON PATIENTEN UND KÜHLEINRICHTUNG MIT EINER SOLCHEN AUFLAGE**
COVER FOR COOLING PATIENTS AND COOLING DEVICE COMPRISING A COVER OF THIS TYPE
REVETEMENT POUR REFROIDIR DES PATIENTS ET DISPOSITIF DE REFROIDISSEMENT DOTE D'UN REVETEMENT DE CE TYPE

(30) Priorität: 01.10.2004 AT 16432004
(43) Veröffentlichungstag der Anmeldung: 13.06.2007
(73) Patentinhaber: Emcools-Emergency Medical cooling systems AG, 1200 Vienna (AT)
(72) Erfinder: BEHRINGER, Wilhelm, A-1090 Wien (AT); STERZ, Fritz, A-1160 Wien (AT); FAWORKA, Rudolf, A-1110 Wien (AT)
(74) Vertreter: Sonn & Partner Patentanwälte
(86) Internationale Anmeldenummer: PCT/AT2005/000324
(87) Internationale Veröffentlichungsnummer: WO 2006/037136

(56) Entgegenhaltungen:
- EP-A- 0 046 894
- EP-A- 0 342 676
- DE-A1- 4 005 718
- US-A- 2 208 855
- US-A- 2 563 933
- US-A- 5 800 491
- US-A- 5 837 002
- US-B1- 6 610 084

## Beschreibung

Die Erfindung betrifft eine Auflage zum Kühlen zumindest von Teilen des Körpers von Patienten, mit zumindest einem eine Kühlflüssigkeit enthaltenden Kühlelement zur Auflage auf den Körper oder Körperteil, welches Kühlelement vor der Anwendung unter den Gefrierpunkt abgekühlt wird.

Weiters betrifft die Erfindung eine Einrichtung zum Kühlen zumindest von Teilen des Körpers von Patienten, mit zumindest einer oben beschriebenen Kühlauflage und einem Kühlgerät.

Die vorliegende Erfindung bezieht sich insbesondere auf die Kühlung von Herzstillstand- oder Schlaganfallpatienten. Nichtsdestotrotz ist eine Anwendung auch bei Patienten nach einen Gehirntrauma, Rückenmarkstrauma oder septischem Schock möglich. Schließlich kann die gegenständliche Kühlauflage auch zum Kühlen von Verletzungen, Verstauchungen usw. verwendet werden. Schließlich kann die gegenständliche Kühlauflage auch zum Kühlen von Produkten, beispielsweise von Lebensmitteln oder dgl. verwendet werden.

Untersuchungen haben gezeigt, dass die Überlebenschance von Patienten mit Herzstillstand durch Reduktion der Körpertemperatur nach erfolgreicher Wiederbelebung wesentlich erhöht werden kann. Durch die Anwendung einer Hypothermie wird nicht nur der Sauerstoffverbrauch im Gehirn des Patienten reduziert, sondern es werden verschiedene zelluläre Zerfallsprozesse verlangsamt, durch die selbst nach erfolgreicher Wiederherstellung des Kreislaufes irreparable neurologische Schäden auftreten. Trotz fortschrittlicher Ambulanz- und Notfallversorgung und den Einsatz von neusten medizinischen Technologien in der Intensivmedizin sind die Chancen eines Patienten einen Herzstillstand außerhalb eines Krankenhauses zu überleben nach wie vor sehr gering. Die Inzidenz von plötzlichem Herzstillstand außerhalb des Krankenhauses liegt in Industrieländern zwischen 36 und 128 pro 100.000 Einwohnern pro Jahr. Wenn die Opfer eines Herz-Stillstands innerhalb der ersten 4-6 Minuten keine Behandlung erhalten, können bereits irreversible Hirnschäden eintreten. Die Chancen auf Überleben verringern sich mit jeder Minute ohne Behandlung um 7 bis 10%. Nach 10 Minuten sind nur mehr wenige Reanimationsversuche erfolgreich.

Die derzeitige Therapie nach einem Herzstillstand konzentriert sich auf Wiederbelebungsmaßnahmen. Die Verwendung von lebensunterstützenden medizinischen Geräten und von hoch entwickelten Operationstechniken ermöglicht es Ärzten, den Kreislauf der Opfer auch nach längeren Stillstand-Zeiten wieder herzustellen, mit dem Problem von irreversiblen Schädigungen des Gehirns. Auch nach der Wiederherstellung der spontanen Zirkulation schreitet der fatale Schädigungsprozess von Gehirn und Organen aufgrund chemischer und physischer Veränderungen des Blutes während des Herzstillstands fort (Post-Reanimations-Syndrom). Die für die Schädigung des Gehirns verantwortlichen pathophysiologischen Mechanismen vor, während und nach der Reanimation sind vielfältig. Bis jetzt gibt es keine spezifische Therapie, um das Gehirn nach der Wiederherstellung der spontanen Zirkulation zu schützen. Biomedizinische Pharmazeutika, welche die Mechanismen der zellulären Zerstörung behindern können, stellen ein viel versprechendes Forschungsfeld dar, das sich jedoch noch in den Anfängen befindet. Forschungsgruppen auf der ganzen Welt untersuchen daher andere Möglichkeiten, um diese fatalen Mechanismen zu bewältigen.

Zur Zeit stellt die Hypothermie das fortschrittlichste medizinische Konzept zur Vorbeugung oder Linderung des Post-Reanimations-Syndroms dar. Viele Studien zeigen einen ausgeprägten positiven Effekt der Hypothermie nach spezifischen ischämischen Zuständen, insbesondere nach dem Herzstillstand. Im Gegensatz zur unkontrollierten Hypothermie macht die therapeutische Hypothermie, wie sie für die Herz- und Neurochirugie oder für die Reanimation nach einem Herzstillstand zum Einsatz kommt, kontrollierte Bedingungen erforderlich. In der therapeutischen Hypothermie werden verschiedene Grade der Kühlung definiert:
Milde Hypothermie: 36-33°C
Moderate Hypothermie: 32-28°C
Tiefe Hypothermie: 27-11°C
Profunde Hypothermie: 10-6°C
Ultra profunde Hypothermie: 5-0°C

Studien, welche die Anwendung der milden Hypothermie in Vergleich zu Normothermie in komatösen Überlebenden eines Herzstillstands kardiologischer Ursache untersuchen, zeigen, dass die Herabstetzung der Körpertemperatur die Überlebensrate und die neurologische Erholung in diesen Patienten verbessert. Von der American Heart Association wurde im Juli 2003 die Empfehlung abgegeben, Opfer eines Herzstillstands außerhalb eines Krankenhauses mittels milder Hypothermie zu kühlen. Diese Empfehlung wurde in Europa bereits im Oktober 2002 von der ILOR (International Liaison commitee of Resuscitation), welcher das ERC ( European Resuscitation Council), die AHA (American Heart Association) und viele andere weltweite Vereinigungen angehören und die sich um die Erarbeitung von einheitlichen Richtlinien für die Cardiopulmonale Reanimation (CPR) bemühen, abgegeben.

Bei der milden Hypothermie ist der Zeitpunkt des Beginns der Kühlung und deren Dauer von entscheidender Bedeutung. Die derzeit zur Verfügung stehenden Kühlungsmethoden sind für eine frühe Induktion von Hypothermie nicht geeignet. Das Eintauchen in Eiswasser führt zwar zu relativ schneller Kühlung, ist aber praktisch nicht durchführbar. Bei der Entfernung von Kleidung und der Anbringung von Eispackungen an Kopf und Torso tritt die Kühlung zu langsam ein. Das extrakorporale Kühlen von Blut ist zwar die schnellste Methode zur Reduktion der Temperatur, bringt aber logistische Schwierigkeiten mit sich. Obwohl die Verwendung eines kardiopulmonalen Bypasses und eines Wärmeaustauschers zu einer schnellen Reduktion der Temperatur führt, wird die Kühlung um jene Zeit verzögert, die benötigt wird, um einen vaskulären Zugang zu erhalten und die Geräte vorzubereiten. Auch die intravenöse Infusion großer Volumina eiskalter Flüssigkeit führt nur zu einer langsamen Abkühlung des Patienten.

Milde Hypothermie sollte so schnell wie möglich nach erfolgreicher Wiederbelebung eingeleitet werden. Im Gegensatz zu milder Hypothermie nach erfolgreicher Wiederbelebung zeigt sich tierexperimentell ein anderer Einsatz der Hypothermie als vielversprechend, nämlich die sehr rasche Induktion tiefer Hypothermie bereits im Herzstillstand, um dann den Patienten unter dem Schutz der Kühlung in das Krankenhaus zu transportieren, und erst im Krankenhaus unter kontrollierten Bedingungen wieder zu beleben ("Suspended Animation"). Dieses Konzept muss aber erst in Tierexperimenten bewiesen werden.

Eine schnell induzierte hypotherme Kühlung, welche den Mechanismus der zellulären Zerstörung unterbindet, ist nicht auf Herzstillstands-Opfer beschränkt. Andere mögliche Indikationen, bei welchen sich die Herabsetzung der Körpertemperatur als günstig erwiesen hat sind beispielsweise Herzinfarkt, Apoplexie, Hirntrauma, Rückenmarksverletzungen oder septischer Schock.

Momentan erhältliche, nichtinvasive Kühlungsvorrichtungen sind nicht in der Lage den Patienten rasch abzukühlen, da die niedrige Temperatur durch Haut und Muskel transportiert werden muss und diese Systeme nur partiell und nicht über die gesamte Körperoberfläche wirken. Darüber hinaus sind bestehende Geräte sehr groß, schwer und kompliziert handzuhaben und benötigen eine relativ lange Vorbereitungszeit. Darüber hinaus erfordern die verfügbaren Geräte meist eine permanente elektrische Versorgung, die beispielsweise in einem Rettungsfahrzeug nicht zur Verfügung steht.

Die US 2002/0193852 A1 beschreibt ein leichtes tragbares System zur Erwärmung oder Kühlung von Patienten, umfassend eine Einrichtung zur Bereitstellung eines flüssigen Kühlmediums und eine vom Kühlmedium durchströmte Einrichtung zur Abgabe der vom Kühlmedium transportierten Kälte an den Patienten. Die Abgabeeinrichtung wird um den Patienten angeordnet, wobei das Gesicht des Patienten ausgespart bleibt. Die sackförmige Abgabeeinrichtung beinhaltet Abstandelemente, zwischen welche Hohlräume zur Führung des Kühlmediums gebildet sind. Am Ende des Sackes wird die gekühlte bzw. erwärmte Flüssigkeit eingeströmt und auf der gegenüberliegenden Seite abgeleitet. Abgesehen von den auftretenden Dichtheitsproblemen ist die beschriebene Einrichtung aufgrund der hohen benötigen Flüssigkeitsmengen sehr voluminös und schwer. Darüber hinaus können mit dieser Methode nur relativ niedrige Kühlraten erzielt werden. Schließlich wird der Kopf des Patienten unzureichend von der Kühlflüssigkeit umströmt und damit unzureichend gekühlt. Ferner kann durch die Umhüllung des Patienten an diesen keine Untersuchung oder Therapie, wie z.B. Herzmassage durchgeführt werden.

Weitere bekannte Kühlvorrichtungen haben den Nachteil, dass die Haut häufig auf Temperaturen unter 0°C abgekühlt wird, wodurch es zu Verbrennungen an der Haut kommt. Beispielsweise ist die Kühlung von Körperteilen mit Hilfe von Eiswürfeln oder Kühlbeuteln, welche ein Kühlmedium mit einem Gefrierpunkt unter 0°C (wie zum Beispiel gefrorene Salze, Alkohollösungen oder Gase) enthalten und im Tiefkühlschrank aufbewahrt werden gefährlich, da bei direkter Auflage des Kühlelements die Haut auf Temperaturen unter 0°C abgekühlt werden und es zu Verletzungen kommen kann. Bei der Kühlung, beispielsweise mit Eiswürfeln, besteht das Problem darin, dass sich auf Grund des schmelzenden Eises eine Isolationsschicht aus Wasser zwischen Körperoberfläche und dem Eiswürfel bildet. Auf Grund der schlechten Wärmeleitfähigkeit von Wasser ist eine optimale Kühlung des Körpers nicht möglich.

Ausgehend von diesem Stand der Technik liegt der vorliegenden Erfindung die Aufgabe zugrunde, eine Auflage zum Kühlen zumindest von Teilen des Körpers von Patienten, insbesondere mit Herzstillstand der oben angegebenen Art zu schaffen, mit der möglichst rasche Kühlraten erzielt werden können ohne dass der Patient durch zu tiefe Temperaturen Schaden erleiden kann. Die Kühlauflage soll möglichst klein und leicht sein, so dass ein Einsatz auch außerhalb des Krankenhauses beispielsweise in Rettungsfahrzeugen aber auch außerhalb dieser Einrichtung ermöglicht wird. Die Kühlauflage soll von nicht speziell ausgebildetem Personal angewendet werden können. Zudem soll die Kühlauflage möglichst kostengünstig herstellbar sein, so dass Sie auch als Einwegprodukt verwendet werden kann.

Eine weitere Aufgabe der vorliegenden Erfindung besteht in der Schaffung einer oben genannten Einrichtung zum Kühlen zumindest von Teilen des Körpers von Patienten, mit zumindest einer oben genannten Kühlauflage und einem Kühlgerät, mit der möglichst rasche Kühlraten erzielt werden können und welche möglichst klein und leicht ausgebildet ist. Die Kühleinrichtung soll möglichst unabhängig von externer elektrischer Versorgung anwendbar sein, so dass ein Einsatz auch außerhalb von Krankenhäusern oder Rettungsfahrzeugen möglich ist.

Nachteile bekannter Systeme sollen vermieden bzw. reduziert werden.

Die erste erfindungsgemäße Aufgabe wird dadurch gelöst, dass innerhalb des Kühlelements ein im Vergleich zur Kühlflüssigkeit thermisch gut leitfähiges Material zur Aufnahme der Kühlflüssigkeit enthalten ist. Durch dieses Merkmal wird die üblicher Weise schlechte Wärmeleitfähigkeit der Kühlflüssigkeit, beispielsweise von Wasser überbrückt, und durch die gute Leitfähigkeit erreicht, dass nach Aufbringen der Kühlauflage auf die Haut des Patienten sehr schnell die Schmelztemperatur der Kühlflüssigkeit erreicht wird. Somit kann die große Schmelzwärme des Eises zu den Kühlzwecken verwendet werden. Unter der Voraussetzung der Wahl entsprechender Kühlflüssigkeiten werden auf diese Weise Erfrierungen an der Haut vermieden. Sofern durch die Kühlauflage eine entsprechende Wärmekapazität geschaffen wird, kann eine besonders rasche Abkühlung des Körpers lediglich durch Auflegen der Kühlauflage erreicht werden. Durch die erfindungsgemäße Kombination der Verwendung einer Kühlflüssigkeit, insbesondere von Wasser, welches in einem Material mit verleichsweise guter Wärmeleitfähigkeit enthalten ist, können die gewünschten raschen Kühlraten erzielt werden. Notwendig dafür ist, dass die Wärmekapazität der Kühlauflage entsprechend groß ist, um die Abkühlung des Körpers bzw. des Körperteiles des Patienten zu erzielen. Dabei wird die Schmelzwärme von Eis, das ist jene Wärme, die das Eis aufnimmt um flüssig zu werden, für die Kühlung des Körpers ausgenützt. Durch das Material mit der guten Wärmeleitfähigkeit wird verhindert, dass sich eine isolierende Wasserschicht bildet, welche eine weitere Abkühlung des Körpers oder Körperteils des Patienten verhindert. Ein Vorteil gegenüber anderen bekannten Systemen ist, dass die Anwendung der Kühlauflage besonders einfach ist und somit auch von ungeschultem Personal vorgenommen werden kann, und zur Durchführung von Untersuchungen oder Therapien (wie z.B. Herzmassage) die Auflage auch kurzzeitig abgehoben werden kann. Schließlich ist eine Fehlindikation mit der erfindungsgemäßen Kühlauflage auch eher unwahrscheinlich, da die Kühlauflage durch die erfindungsgemäße Kombination einer guten Wärmeleitfähigkeit und hoher Wärmekapazität keinen Schaden an der Haut und auch weniger leicht an den inneren Organen bewirken kann. Die Kühlauflage kann je nach Anwendungsgebiet verschieden groß und verschieden dick ausgeführt sein.

Das thermisch gut leitfähige Material kann dabei durch Metallwolle aus einem Metall oder einer Metalllegierung mit guter Wärmeleitfähigkeit, beispielsweise Aluminium, Kupfer oder Stahl gebildet sein. Die Metallwolle ist in jedem Kühlelement von einer entsprechenden Umhüllung umgeben und mit der Kühlflüssigkeit getränkt. Nach Abkühlen der Kühlauflage, beispielsweise in einem Gefrierschrank, nimmt das flüssige Kühlmedium das die Metallwolle durchdringt, festen Zustand an. Bei der Anwendung der Kühlauflage wird die an sich schlechte Wärmeleitfähigkeit der Kühlflüssigkeit durch die Metallwolle verbessert und somit ein rascher Wärme- bzw. Kälteübergang von der Kühlauflage zum Körper und somit eine rasche Absenkung der Temperatur an der Hautoberfläche auf die Schmelztemperatur der Kühlflüssigkeit erreicht. Liegt die Schmelztemperatur der Kühlflüssigkeit nicht wesentlich unter 0°C, sind keine Verbrennungen der Haut zu befürchten.

Ebenso ist es möglich, das thermisch gut leitfähige Material durch Metallschaum aus einem Metall oder einer Metalllegierung mit hoher Wärmeleitfähigkeit, beispielsweise Aluminium, Kupfer oder Stahl, zu bilden. Bei Metallschaum handelt es sich um einen Werkstoff aus Metall, der besonders geringes Gewicht und hohe mechanische Stabilität aufweist. Zudem ist aufgrund der Poren im Metallschaum eine Durchströmbarkeit mit einer Flüssigkeit möglich und eine große innere Oberfläche gegeben.

Dabei ist der Metallschaum vorzugsweise offenporig ausgebildet, so dass möglichst viel Kühlflüssigkeit aufgenommen werden kann.

Weiters ist es möglich, als thermisch gut leitfähiges Material Graphiteinzusetzen. Graphit hat gegenüber den oben genannten Metallen eine höhere Wärmeleitfähigkeit und ist zudem leichter. Weiters ist dieser Werkstoff auch billiger und biologisch unbedenklich. Dabei kann auch Graphit in Form so genannten Blähgraphits zum Einsatz kommen. Graphit hat ein enormes Aufnahmevermögen von Flüssigkeit. Beispielsweise können mit Graphit ausgefüllte Volumen bis zu 90 % mit Wasser befüllt werden. Dadurch eignet sich dieser Werkstoff im besonderen Maße für die erfindungsgemäße Anwendung.

Um eine Verletzung der Haut des Patienten durch zu niedrige Temperaturen sicher zu vermeiden, wird die Kühlflüssigkeit durch Wasser gebildet. Da Wasser einen Schmelzpunkt von 0°C aufweist, kann es nicht zu Temperaturen unter 0°C an der Haut und somit nicht zu Verbrennungen der Haut kommen. Dabei wird vorzugsweise Reinstwasser verwendet. Auch ist die Schmelzwärme bei Wasser mit 335 kJ/kg relativ hoch. Die Schmelzwärme ist jene Wärme, die das Eis aufnimmt, um flüssig zu werden.

Zur Erzielung einer flexibel anwendbaren Kühlauflage sind vorteilhafter Weise mehrere Kühlelemente auf einer flexiblen Unterlage angeordnet. Unter der Voraussetzung einer geeigneten Wahl der Dimension für die Kühlelemente wird dadurch eine optimale Anpassung der Kühlauflage an die unterschiedlichen Oberflächen der zu kühlenden Körperteile erzielt.

Die flexible Unterlage ist dabei vorzugsweise aus Latex gebildet. Dieses aus Naturkautschuk hergestellte Material ist besonders leicht verarbeitbar, relativ billig und weist eine enorme Dehnfähigkeit auf. Darüber hinaus ist das Material umweltverträglich und verrottbar und hält die niedrigen Temperaturen aus.

Die flexible Unterlage kann auch aus Silikon gebildet. Dieses Material ist besonders flexibel und dehnfähig, so dass die Kühlauflage leicht auf die Haut aufgelegt werden kann.

Gemäß einem weiteren Merkmal der Erfindung ist eine wärmeisolierende Schicht zur Anordnung zwischen den Kühlelementen oder der Unterlage und dem Körper oder Körperteil vorgesehen. Dadurch kann ein besserer Schutz der Hautoberfläche vor Unterkühlungen erreicht werden, was bei verschiedenen Anwendungsgebieten zweckmäßig sein kann. Natürlich kann die wärmeisolierende Schicht gleich an der Kühlauflage befestigt oder mit dieser einstückig hergestellt sein.

Um ein Reißen der Unterlage zu verhindern, kann diese eine Verstärkungsschicht, beispielsweise aus einem Gewebe, beinhalten.

Zur Erzielung einer möglichst hohen Wärmekapazität des Kühlelementes bei gleichzeitig geringen Abmessungen desselben, insbesondere geringer Höhe, ist das zumindest eine Kühlelement vorzugsweise im Wesentlichen quaderförmig ausgebildet.

Um eine rasche Erwärmung der Kühlauflage durch die Umgebungsluft zu verhindern, kann an der dem Körper abgewandten Seite des zumindest einen Kühlelements eine Wärmeisolierung angeordnet sein. Eine derartige Isolierung kann durch verschiedene Materialien mit schlechter Wärmeleitfähigkeit, die sich leicht verarbeiten lassen, erzielt werden.

Zusätzlich kann an der dem Körper abgewandten Seite des zumindest einen Kühlelements eine Reflexionsschicht angeordnet sein, so dass eine Erwärmung der Kühlauflage, beispielsweise durch Sonneneinstrahlung vermieden bzw. reduziert wird.

Die Kühlelemente können wie die Unterlage aus Latex gebildet sein. Dieses Material ist, wie bereits oben erwähnt, besonders leicht verarbeitbar, relativ billig und weist gute Dehnungsfähigkeit auf.

Die Kühlelemente können auch aus Silikon gebildet sein. Dieses Material weist, wie bereits oben erwähnt, besonders hohe Flexibiltät und Dehnungsfähigkeit auf.

An der dem Körper zugewandten Fläche des Kühlelements kann eine Platte aus einem Material mit besonders hoher Wärmeleitfähigkeit angeordnet sein um so den Übergang der Kälte zum Patienten zu fördern. Dabei kann es sich um eine Platte aus Metall handeln.

Alternativ dazu kann die Unterlage zumindest an den Stellen unterhalb der Kühlelemente mit geringerer Dicke ausgebildet sein, um einen optimalen Wärmeübergang zu schaffen.

Um eine flexible Gestaltung der Kühlauflage zu erreichen, können an dem zumindest einen Kühlelement oder der Unterlage Element zur Verbindung mit anderen Kühlelementen oder Unterlagen vorgesehen sein. Dadurch können mehrere Kühlelemente modular aneinandergereiht und miteinander verbunden werden. Die Größe und Gestalt der resultierenden Kühlauflage wird an den jeweiligen Einsatzfall angepasst.

Die Verbindungselemente können durch Reißverschlüsse gebildet sein.

Um ein Verrutschen der Kühlauflage am Patienten zu verhindern, kann an dem zumindest einen Kühlelement oder der Unterlage ein Element zur Fixierung am Patienten, beispielsweise ein Gurt mit einem Schnellverschluss, wie einem Klettverschluss, vorgesehen sein. Wenn dieses Fixierelement gleich an der Kühlauflage befestigt ist, wird sicher erreicht, dass das Fixierelement im Einsatzfall zur Hand ist. Dies ist insbesondere bei der Anwendung bei Herzstillstandpatienten von besonderer Bedeutung, da hier Rettungsmaßnahmen besonders schnell erfolgen müssen.

Für einen besseren Kontakt der Kühlauflage am Patienten kann auch an der dem Körper zugewandten Fläche des Kühlelements eine Klebeschicht angeordnet sein. Vor der Anwendung der Kühlauflage wird vorzugsweise eine Abdeckfolie, welche über der Klebeschicht andeordnet ist, abgezogen und danach die Kühlauflage auf die Haut des Patienten aufgeklebt. Dabei werden vorzugsweise hautverträgliche Kleber verwendet. Die Klebeschicht kann zumindest auf Teile der Unterseite der Kühlauflage beispielsweise in flüssigem Zustand aufgebracht und danach mit einer entsprechenden Folie abgedeckt werden. Ebenso ist es möglich, die Klebeschicht in Form von doppelseitigen Klebebändern an die dem Körper zugewandten Seite der Kühlauflage anzubringen.

Wenn zwischen den Kühlelementen der Kühlauflage Einschnitte, Perforationen oder dgl. angeordnet sind, kann die Kühlauflage besonders einfach und vorzugsweise werkzeuglos getrennt und den entsprechenden Erfordernissen der Größe nach angepasst werden.

Gemäß einem weiteren Merkmal der Erfindung können Sensoren zur Messung der Temperatur des Patienten vorgesehen sein. Durch diese Sensoren, welche auch mit einer entsprechenden Elektronik und mit entsprechenden akustischen oder visuellen Ausgabeeinrichtungen verbunden sein können, kann beispielsweise eine Überwachung der Temperaturen an der Hautoberfläche vorgenommen werden und aufgrund der ermittelten Temperaturwerte bestimmte Schritte gesetzt werden. Auch die Überwachung der Temperatur in der kernnahen Region des Körpers ist besonders wichtig, da beispielsweise im Falle der Abkühlung des Herzmuskels unter 30°C wiederum die Gefahr eines Herzstillstandes gegeben ist.

Im Falle der Verwendung der Kühlauflage bei Herzstillstandpatienten ist es von Vorteil, wenn eine elektrische Einrichtung zur Herzmassage angeordnet ist. Auf diese Weise kann eine Kombination der Kühlung des Patienten bei gleichzeitiger automatischer Herzmassage erreicht werden. Automatische Pumpen zur Herzmassage werden um den Brustkorb des Patienten geschnallt. Durch periodische Druckimpulse auf den Brustkorb wird der Blutkreislauf aufrechterhalten. Wenn nun ein derartiges automatisches Herzmassagegerät auch mit der erfindungsgemäßen Kühlauflage kombiniert wird, steigen die Überlebenschancen des Patienten noch weiter.

Die Kühlelemente können je nach Einsatzgebiet in Form einer Decke oder eines Schlafsackes angeordnet sein.

Zur Kühlung des Gehirns kann auch die Form einer Kopfbedeckung gewählt werden. Dabei muss natürlich auch die Größe der Kühlelemente entsprechend angepasst werden. Für eine Kopfbedeckung werden zur Erzielung kleinerer Biegeradien der Kühlauflage kleinere Kühlelemente als für eine Decke verwendet werden.

Ebenso ist es möglich, die Kühlelemente in Form von Schläuchen zur Aufnahme der Arme oder Beine des Patienten anzuordnen.

Weiters können die Kühlelemente in Form eines Fäustlings oder eines Strumpfes zur Anwendung, beispielsweise bei Verstauchungen der Händer oder Füße angeordnet sein.

Bei Verwendung verschiedener Kühlflüssigkeiten oder auch verschiedener Größen von Kühlelementen kann zur Unterstützung bei der Auswahl der entsprechenden Kühlauflage eine Codierung, vorzugsweise einer Farbcodierung, vorgesehen sein. Dadurch kann beispielsweise der Arzt oder Sanitäter rasch die entsprechende Kühlauflage auswählen und anwenden.

Die zweite erfindungsgemäße Aufgabe wird durch eine oben genannte Einrichtung gelöst, wobei das Kühlgerät zur Abkühlung der Kühlauflage auf Temperaturen unter 0°C ausgebildet ist. Dabei ist es lediglich entscheidend, die Kühlflüssigkeit einzufrieren, um für den Kühlvorgang die Schmelzwärme, welche aufgenommen wird, wenn die Kühlflüssigkeit vom gefrorenen in den flüssigen Zustand übergeht, auszunützen. Abkühlungen weiter unterhalb dem Gefrierpunkt bringen für die Gesamtbilanz nur noch wenig.

Das Kühlgerät kann durch ein elektrisch angetriebenes Kühlaggregat in Art eines Tiefkühlgeräts gebildet sein.

Ebenso ist es möglich, dass das Kühlgerät durch ein Peltier-Element gebildet ist.

Für Ambulanzfahrzeuge oder bei anderen Anwendungsfällen kann es von Vorteil sein, dass das Kühlgerät ohne externe Energiequelle auskommt und lediglich durch einen passiven Behälter mit einer Wärmeisolierung zur Aufnahme der Kühlauflage gebildet ist. Dabei wird die Kühlauflage zuerst in einem Tiefkühlgerät abgekühlt und dann für eine bestimmte Zeit in den genannten passiven Behälter mit der Wärmeisolierung aufbewahrt werden. Durch Wahl einer entsprechenden Wärmeisolierung können ohne Zuführung externer Energien Zeiten von einigen Tagen bis zu einer Woche erreicht werden, während der es zu keiner Erwärmung der Kühlauflagen kommt, welche eine Anwendung derselben nicht mehr möglich machen.

Dabei ist eine Wärmeisolierung des Behälters aus evakuierter Kieselsäure besonders effizient. Beispielsweise bietet die Firma Wacker unter der Bezeichung WDS^{®}, Wärmedämmfolien aus Kieselsäure an, welche hervorragende Isoliereigenschaften aufweisen.

Für die Anwendung in Rettungsfahrzeugen, aber auch in Ambulanzbereichen ist es von Vorteil, wenn das Kühlgerät in einer Patientenliege integriert ist. Somit ist die Kühlauflage jederzeit griffbereit und kann rasch angewendet werden, so dass bei Herzstillstandpatienten eine höhere Überlebenschance erreicht werden kann.

Gemäß einem weiteren Merkmal der Erfindung ist zumindest ein Sensor zur Messung der Temperatur vorgesehen. Durch einen derartigen Sensor, der mit einer Auswertungseinheit und allenfalls einer akustischen und bzw. oder visuellen Ausgabeeinheit verbunden sein kann, ist es möglich die Temperatur beispielsweise im Kühlgerät zu dokumentieren und beispielsweise bei Überschreiten einer bestimmten Temperatur bestimmte Maßnahmen ergriffen werden.

Abschließend sei erwähnt, dass die erfindungsgemäße Kühlauflage und Kühleinrichtung nicht nur bei Menschen, sondern theoretisch auch bei Tieren angwendet werden kann.

Die vorliegende Erfindung wird anhand der beigefügten Zeichnungen näher erläutert.

Darin zeigen:
Fig. 1 eine schematische Ansicht auf einen Patienten mit daran angeordneten Kühlauflagen;
Fig. 2 einen Schnitt durch den Patienten entlang der Schnittlinie II-II aus Fig. 1;
Fig. 3 eine Draufsicht auf ein Kühlelement einer Kühlauflage;
Fig. 4 einen Schnitt entlang der Schnittlinie IV-IV durch das Kühlelement gemäß Fig. 3;
Fig. 5 einen Schnitt durch einen Teil einer Kühlauflage in vergrößerten Maßstab;
Fig. 6 die Draufsicht auf eine aus mehreren Elementen aufgebaute Kühlauflage;
Fig. 7 einen Schnitt durch eine Kühleinrichtung zur Kühlung einer Kühlauflage und
Fig. 8 die Temperaturverläufe an der Haut und unterhalb der Haut aus einem Tierexperiment.

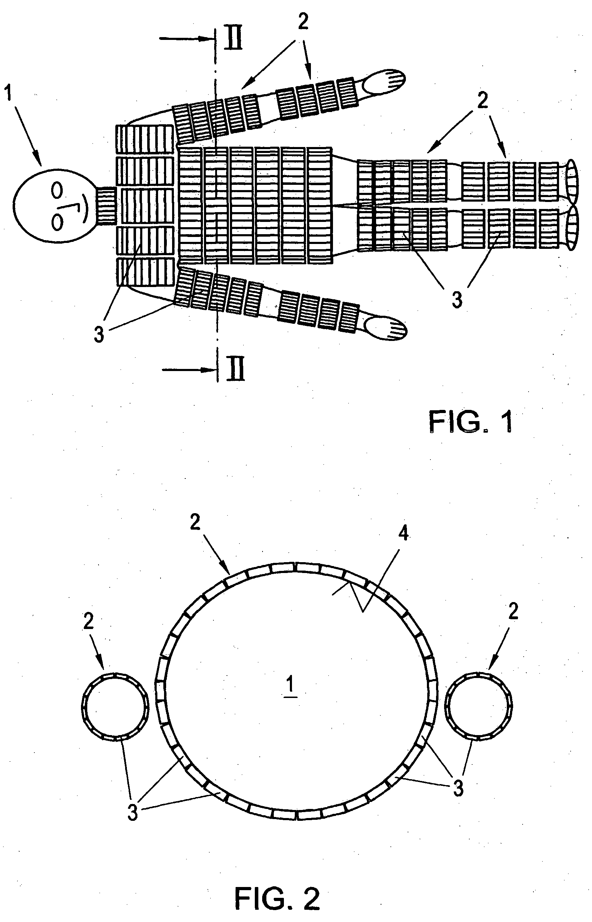

Fig. 1 zeigt eine schematische Draufsicht auf einen Patienten 1 , an dem sowohl am Oberkörper als auch an den Extremitäten erfindungsgemäße Kühlauflagen 2 angeordnet sind. Die Kühlauflagen 2 bestehen aus zumindest einem Kühlelement 3, welches in der Folge näher erläutert wird. Je nach Anwendungsfall können die Kühlauflagen 2 flächig oder als Schlauch ausgebildet sein. Die Kühlauflagen 2 sind besonders schnell und einfach anwendbar und verhindern aufgrund der erfindungsgemäßen Merkmale eine Abkühlung der Haut auf zu tiefe Temperaturen und somit das Entstehen von Verbrennungen. Andererseits ist es durch die Kühlauflagen 2 möglich, rasch die Körpertemperatur abzusenken und beispielsweise im Fall eines Herzstillstandes die Überlebenschancen und die Chancen einer vollständigen Genesung zu erhöhen.

Fig. 2 zeigt einen Schnitt entlang der Schnittlinie II-II durch den Patienten 1 gemäß Fig. 1. Dabei sind um den Thorax und die Arme schlauchförmige Kühlauflagen 2 angeordnet. Zur leichteren Anwendung der Kühlauflagen 2 können diese flächig aufgebaut sein und um den Körper oder Körperteil des Patienten 1 herum gelegt und befestigt werden. Bei Herzstillstandpatienten ist es wesentlich den Brustbereich, den Rückenbereich zum Schutz des Rückenmarks und den Kopfbereich zum Schutz des Gehirns mit den Kühlauflagen 2 abzudecken. Die Kühlauflagen 2 bestehen aus vorzugsweise mehreren Kühlelementen 3, die auf einer flexiblen Unterlage 4, beispielsweise aus Latex, angeordnet sind. Anstelle der Verwendung einer Unterlage 4 können natürlich die Kühlelemente 3 auch miteinander verbunden sein.

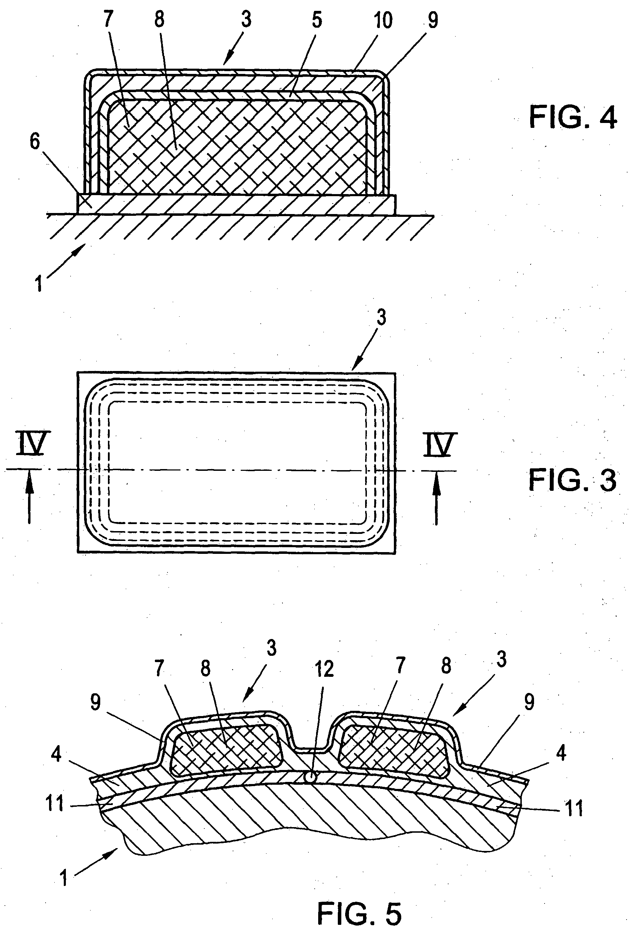

Fig. 3 zeigt eine Draufsicht auf ein Kühlelement 3, welches beispielsweise quaderförmige Gestalt aufweist. Wie aus der Schnittansicht gemäß Fig. 4 ersichtlich ist, besteht das Kühlelement 3 aus einer Umhüllung 5, welche aus kältebeständigen dehnungsfähigem Kunststoff, beispielsweise Latex oder auch Silikon, besteht. Die Umhüllung 5 ist mit einer Kontaktplatte 6 verbunden, welche vorzugsweise aus wärmeleitfähigem Material, wie z.B. Metall oder wärmeleitfähigem Kunststoff besteht. Selbstverständlich kann die Umhüllung 5 und die Kontaktplatte 6 auch einstückig hergestellt sein. Dabei eignet sich Latex besonders, da es sehr leicht verarbeitbar ist. Darüber hinaus ist dieses Material umweltverträglich und hält die niedrigen Temperaturen aus, ohne dass die Eigenschaften verschlechtert werden. Im Kühlelement 3 befindet sich ein thermisch gut leitfähiges Material 7, in dem die Kühlflüssigkeit 8 eingebettet ist. Durch das thermisch gut leitfähige Material 7, welches beispielsweise durch Metallwolle, einem Metallschaum oder Graphit gebildet sein kann, wird die Wärmeleitfähigkeit erhöht und somit die Kälte der Kühlflüssigkeit 8 rascher an die Oberfläche des Körpers des Patienten 1 transportiert. Um ein Erwärmen der Kühlflüssigkeit 8 innerhalb des Kühlelements 3 von außen zu verhindern bzw. zu reduzieren, kann an der dem Körper des Patienten 1 abgewandten Seite des Kühlelements 3 eine Wärmeisolierung 9 angeordnet sein. Zusätzlich kann an der Wärmeisolierung 9 auch eine Reflexionsschicht 10 zur Verhinderung einer Erwärmung, beispielsweise durch Sonneneinstrahlung, vorgesehen sein. Diese Reflexionsschicht 10 kann beispielsweise durch Aufbringen einer Mischung von Latex mit Aluminiumpartikel hergestellt werden, welche einfach auf die Kühlauflage 2 aufgespritzt werden. Das Kühlelement 3 bzw. eine Anordnung mehrerer Kühlelemente 3 auf einer Unterlage 4, wird auf die jeweilige Körperregion des Patienten 1 aufgelegt. Aufgrund der guten Wärmeleitfähigkeit des Materials 8 innerhalb des Kühlelements 3 kommt es zu einem raschen Abkühlen der Hautoberfläche des Patienten 1 und somit auch zu einer relativ raschen Absenkung der Kerntemperatur des Patienten 1.

Fig. 5 zeigt ein Schnittbild durch einen Teil einer Kühlauflage 2, bei der die Kühlelemente 3 auf einer flexiblen Unterlage 4 angeordnet sind. Dabei sind die Kühlelemente 3 nicht quaderförmig sondern in Form von Pyramidenstümpfen angeordnet, was eine leichtere Herstellbarkeit und bessere Stabilität mit sich bringt. Die Kühlelemente 3 können auch einstückig mit der flexiblen Unterlage 4 hergestellt werden. Im Inneren der Kühlelemente 3 befindet sich das thermisch gut leitfähige Material 8 und die Kühlflüssigkeit 7. Um den Wärmeübergang vom Patienten 1 zum Kühlelement 3 zu verbessern, ist die Unterlage 4 im Bereich der Kühlelemente 3 vorzugsweise mit geringerer Dicke als die übrigen Bereiche ausgeführt. Ebenso ist es natürlich möglich, an der dem Körper des Patienten 1 zugewandten Seite der Kühlelemente 3 eine Kontaktplatte 6 (s. Fig. 4) anzuordnen. Für bestimmte Anwendungen kann zwischen der Kühlauflage 2 und der Hautoberfläche des Patienten 1 eine wärmeisolierende Schicht 11 angeordnet werden um eine zu rasche Unterkühlung der Haut des Patienten 1 unter bestimmte Temperaturwerte zu verhindern. Zur Überwachung der Temperatur an der Hautoberfläche des Patienten 1 kann ein Sensor 12 vorgesehen sein, der entweder lose auf die Haut des Patienten 1 aufgelegt oder aufgeklebt wird oder in der wärmeisolierenden Schicht 11 oder in der Unterlage 4 der Kühlauflage 2 angeordnet ist. Der Temperatursensor 12 ist mit einer entsprechenden Auswerteelektronik und allenfalls einer akustischen oder visuellen Ausgabeeinheit verbunden, um dem Arzt oder Sanitäter die jeweilige Temperatur an der Haut anzeigen zu können. Wie bereits oben erwähnt, können zumindest Teile der dem Körper des Patienten 1 zugewandten Seite der Kühlauflage 2 mit einer Klebeschicht (nicht dargestellt) versehen sein, um eine bessere Verbindung mit der Hautoberfläche des Patienten 1 zu ermöglichen.

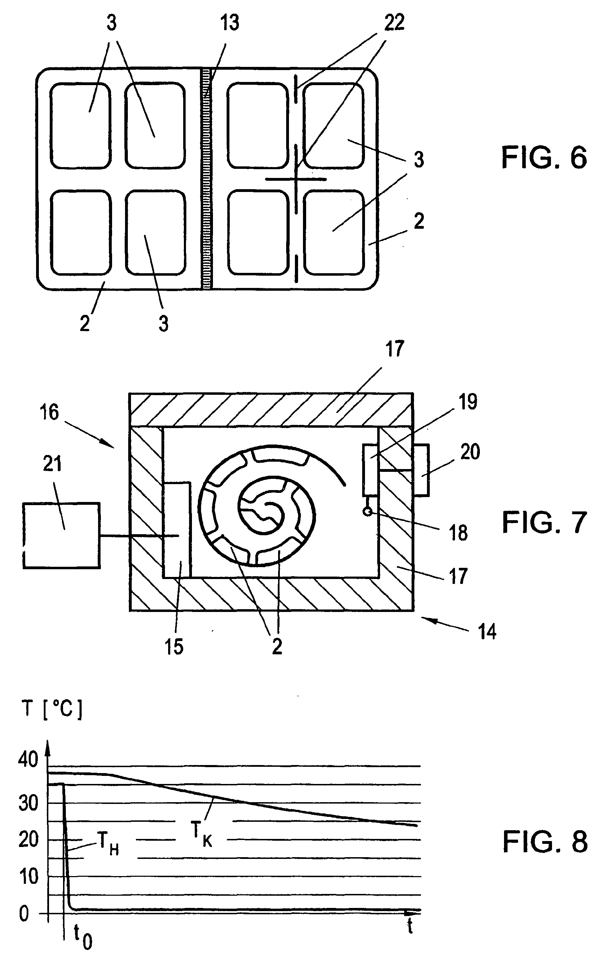

Fig. 6 zeigt eine Draufsicht auf zwei Kühlauflagen 2, bestehend aus jeweils vier Kühlelementen 3, welche mit Verbindungselementen 13, beispielsweise Reißverschlüssen, ausgestattet sind. Auf diese Weise kann aus mehreren Modulen eine geeignete Kühlauflage 2 geschaffen werden. Zwischen den Kühlelementen 3 kann die Kühlauflage 2 auch mit Einschnitten 22, Perforationen oder dgl. versehen sein. Diese verhindern das Entstehen eines isolierenden Luftpolsters zwischen der Hautoberfläche des Patienten 1 und der Kühlauflage 2 und erhöhen andererseits die Flexibilität der Kühlauflage 2. Die Einschnitte 22 können beispielsweise nach Produktion der Kühlauflage 2 durch Stanzen einfach und rasch hergestellt werden. Darüber hinaus kann die Kühlauflage 2 im Bereich derartiger Einschnitte 22 oder Perforationen leichter, vorzugsweise werkzeuglos, getrennt werden und somit die Kühlauflage 2 an die jeweiligen Gegebenheiten der Größe nach angepasst werden.

Optimal ist auch eine Kombination der Kühlauflage 2 mit einer automatischen Einrichtung zur Herzmassage (nicht dargestellt).

Fig. 7 zeigt ein Schnittbild durch ein Kühlgerät 14 zur Kühlung der beschriebenen Kühlauflagen 2 oder zum Schutz der bereits gekühlten Kühlauflagen 2 vor Erwärmung. Das Kühlgerät 14 ist vorzugsweise zur Abkühlung der Kühlauflage 2 auf Temperaturen unter 0°C, bzw. unter dem Gefrierpunkt dieser Kühlflüssigkeit 8 ausgebildet und beinhaltet ein Kühlaggregat 15, welches mit einer elektrischen Versorgung 16 verbunden wird. Das Kühlgerät 14 kann auch durch einen passiven Behälter 21 mit einer Wärmeisolierung 17 zur Aufnahmer der Kühlauflage 2 gebildet sein. Bei Wahl einer entsprechenden Wärmeisolierung 17 kann eine bereits abgekühlte Kühlauflage 2 über mehrere Tage ohne Zufuhr von elektrischer Energie aufbewahrt werden. Im Behälter 16 des Kühlgeräts 14 kann ein Sensor 18 zur Messung der Temperatur vorgesehen sein, der mit einer Auswertungseinheit 19 und allenfalls einer akustischen und bzw. oder visuellen Ausgabeeinheit 20 verbunden sein kann. Dadurch kann die Einsatzbereitschaft der Kühlauflage 2 überwacht werden.

Besonders interessant ist auch eine Anwendung, bei der das Kühlgerät 14 in einer Patientenliege integriert ist. Dadurch können die Kühlauflagen 2 besonders rasch eingesetzt werden was im Falle eines Herzstillstandes des Patienten 1 besonders wichtig ist (nicht dargestellt).

Fig. 8 zeigt schließlich den Verlauf der Temperatur T_{H} an der Hautoberfläche und der Körpertemperatur T_{K} in einer Tiefe von 27,5mm unter der Haut eines Versuchstieres bei der Anwendung einer erfindungsemäßen Kühlauflage 2 in einem Tierexperiment. Dabei wurden Schweine mit einem Gewicht von 75-95kg mit erfindungsgemäßen Kühlauflagen 2 versehen. Die Kühlelemente 3 enthielten reines Wasser, welches in Aluminiumspäne gebettet war. Zum Zeitpunkt t₀ wird die Kühlauflage auf das Versuchstier aufgelegt, worauf sich die Hauttemperatur T_{H} innerhalb weniger Sekunden auf 0°C sinkt. Eine weitere Absenkung der Temperatur unter 0°C ist aufgrund der Verwendung von Wasser als Kühlflüssigkeit 7 nicht möglich. Somit kann es zu keinen Erfrierungen an der Haut des Versuchstieres kommen. Bereits einige Minuten nach dem Applizieren der Kühlauflage 2 zum Zeitpunkt t₀ beginnt die Körpertemperatur T_{K} zu sinken und erreicht schließlich nach etwa 15 Minuten 32-33°C. Die Körpertemperatur T_{K} sinkt je nach Dauer der Anwendung weiter ab und erreicht nach etwa 30 Minuten 24-25°C. Dabei hängt der zeitliche Verlauf der Körpertemperatur T_{K} vom Kreislauf des Versuchstieres bzw. Patienten 1 und von der Größe der Kühlauflage 2 ab. Bei den Tierversuchen an den Schweinen wurde eine Absenkung der Hirntemperatur von 5°C innerhalb von etwa 30 min erzielt. Dabei wurden etwa 0,6m² mit der Kühlauflage 2 abgedeckt.

Die erfindungsgemäße Kühleinrichtung 1 sowie die erfindungsgemäße Abgabeeinrichtung 4 ermöglicht eine besonders rasche Abkühlung von Patienten, insbesondere mit Herzstillstand auch abseits von Krankenhäusern oder dgl. Einrichtungen, wodurch die Überlebenschance erhöht und das Risiko zerebraler Schädigungen erniedrigt werden kann. Die Einrichtung kann auch bei anderen Fällen, wo eine milde oder höhere Hypothermie vorteilhaft ist, angewendet werden.

Anhand eines Beispiels wird die Erfindung noch näher erläutert. Die unten stehende Tabelle zeigt für einige Materialien die Werte bzw. Wertebereiche für die spezifische Wärmekapazität c, die Wärmeleitfähigkeit λ und die Dichte ρ.

| ***Wärmekapazität c*** | | ***Wärmeleitfähigkeit* λ** | ***Dichte (Gewicht)ρ*** |
|---|---|---|---|
| KJ/kg °C | | W/m.K | g/cm³ |
| Aluminium | 0,9 | 230 | 2,71 |
| Graphit | 0,7 | 170 bis 370 | 2,2 |
| Kupfer | 0,38 | 390 | 8,97 |
| Wasser | 4,186 | 0,57 | 1 |
| Eis | 2,1 | 1,7 | |
| Muskelgewebe | 3,6 | 0,36 bis 0,5 | 1 |
| Knochen | 1,2 | 0,5 | 2 |
| Fett | 1,67 | 0,186 bis 0,3 | 0,93 |
| Blut | 4 | 0,472 bis 0,62 | 1 |

Aluminium und Graphit haben ungefähr die gleichen Eigenschaften bezüglich der Wärmeleitfähigkeit λ. Bezüglich Gewicht und Volumen bezogen auf die spezifische Wärmekapazität c hat Graphit Vorteile gegenüber Aluminium. Wasser hat eine sehr schlechte Wärmeleitfähigkeit λ. Wird das Wasser beispielsweise mit 10 Vol.% Aluminium oder Graphit versetzt, so erhöht sich die Wärmeleitfähigkeit λ um etwa das 20-fache. Somit wird durch das Einbringen der Kühlflüssigkeit, insbesondere von Wasser, in ein Material mit demgegenüber sehr guter Wärmeleitfähigkeit λ, die schlechte Wärmeleitfähigkeit des Wassers überbrückt. Die Wärmekapazität von c Eis wird durch das relativ geringe Volumen an Aluminium, Graphit oder Kupfer nicht wesentlich beeinflusst. Somit wird die Wärmekapazität c des Eises mit der Wämeleitfähigkeit λ von Aluminium, Graphit, Kupfer oder dgl. kombiniert. Durch Einfrieren des Wassers auf -5°C bis -20°C entsteht eine Wärmeaufnahmefähigkeit von etwa 10-40 kJ/kg, um die gewünsche Temperatur von 0°C an der Hautoberfläche zu erreichen.

Gemäß einer groben Annahme kann die spezifische Wärmekapazität c von menschlichen Gewebe mit 4 kJ/kg. °C angenommen werden. Bei einer Hauttemperatur von 35°C resultiert eine Wärmeaufnahmefähigkeit von 140 kJ/kg, also 3-14 mal größer als die Wärmeaufnahmfähigkeit der Kühlmatte. Es ist also nicht möglich, dass die Kühlauflage Erfrierungen an der Haut verursacht. Beim Schmelzen des Eises tritt der Kühleffekt durch die Schmelzwärme von Eis auf. Während jedoch bei normalem Eis durch die Wärmeaufnahme und das Schmelzen eine Wasserschicht zwischen der Hautoberfläche und dem Eis entsteht und eine weitere Kühlung des Körpers verhindert, wird durch die vorliegende Erfindung der Aufbau einer Isolierschicht verhindert und somit eine effektive Kühlung erzielt.

Um einen menschlichen Körper mit einem Gewicht von etwa 90kg und einer Körpertemperatur von 37°C um 5°C abzukühlen, ist eine Wärmemenge Q = c.m.ΔT= 4.90.5 = 1800kJ notwendig. Dafür ist eine Masse von etwas mehr als 5kg Eis notwendig. Die theoretischen Werte haben mit den praktischen in Tierexperimenten erprobten Werten gut übereingestimmt. Bei den Studien an Schweinen mit einem Gewicht von 75-95kg wurden jeweils sieben Kühlauflagen mit 14cm x 38cm und sieben Matten stücke mit jeweils 8cm x 30cm und eine Kopfhaube mit 15cm x 40cm verwendet. Dies ergibt eine Oberfläche von etwa 0,6m². Die Kühlauflagen wurden bei -15°C eingefroren. Die erzielten Temperaturabsenkungen um 5°C im Gehirn der Schweine stellten sich innerhalb von 30 min ein.

Die konkreten praktischen Ergebnisse müssen anhand weiterer Experimente ergründet und optimiert werden.

Wie bereits oben erwähnt, kann die Kühlauflage auch zum Kühlen von Produkten, beispielsweise Lebensmitteln oder dgl. eingesetzt werden.

## Patentansprüche

1. Auflage (2) zum Kühlen zumindest von Teilen des Körpers von Patienten (1), mit zumindest einem eine Kühlflüssigkeit (8) enthaltenden Kühlelement (3) zur Auflage auf den Körper oder Körperteil, welches Kühlelement (3) vor der Anwendung unter den Gefrierpunkt abgekühlt wird, **dadurch gekennzeichnet, dass** innerhalb des Kühlelements (3) ein im Vergleich zur Kühlflüssigkeit (8) thermisch gut leitfähiges Material (7) zur Aufnahme der Kühlflüssigkeit (8) enthalten ist.

2. Kühlauflage nach Anspruch 1, **dadurch gekennzeichnet, dass** das thermisch gut leitfähige Material (7) durch Metallwolle aus einem Metall oder einer Metalllegierung mit guter Wärmeleitfähigkeit, beispielsweise Aluminium, Kupfer oder Stahl gebildet ist.

3. Kühlauflage nach Anspruch 1, **dadurch gekennzeichnet, dass** das thermisch gut leitfähige Material (7) durch Metallschaum aus einem Metall oder einer Metalllegierung mit guter Wärmeleitfähigkeit, beispielsweise Aluminium, Kupfer oder Stahl gebildet ist.

4. Kühlauflage nach Anspruch 3, **dadurch gekennzeichnet, dass** der Metallschaum offenporig ausgebildet ist.

5. Kühlauflage nach Anspruch 1, **dadurch gekennzeichnet, dass** das thermisch gut leitfähige Material durch Graphit gebildet ist.

6. Kühlauflage nach einem der Ansprüche 1 bis 5, **dadurch gekennzeichnet, dass** die Kühlflüssigkeit (8) durch Wasser gebildet ist.

7. Kühlauflage nach einem der Ansprüche 1 bis 6, **dadurch gekennzeichnet, dass** mehrere Kühlelemente (3) auf einer flexiblen Unterlage (4) angeordnet sind.

8. Kühlauflage nach Anspruch 7, **dadurch gekennzeichnet, dass** die Unterlage (4) aus Silikon gebildet ist.

9. Kühlauflage nach Anspruch 7, **dadurch gekennzeichnet, dass** die Unterlage (4) aus Latex gebildet ist.

10. Kühlauflage nach einem der Ansprüche 1 bis 9, **dadurch gekennzeichnet, dass** eine wärmeisolierende Schicht (11) zur Anordnung zwischen den Kühlelementen (3) oder der Unterlage (4) und dem Körper oder Körperteil vorgesehen ist.

11. Kühlauflage nach Anspruch 9 oder 10, **dadurch gekennzeichnet, dass** die Unterlage (4) eine Verstärkungsschicht beinhaltet.

12. Kühlauflage nach einem der Ansprüche 1 bis 11, **dadurch gekennzeichnet, dass** das zumindest eine Kühlelement (3) im Wesentlichen quaderförmig ausgebildet ist.

13. Kühlauflage nach einem der Ansprüche 1 bis 12, **dadurch gekennzeichnet, dass** an der, dem Körper abgewandten Seite des zumindest einen Kühlelements (3) eine Wärmeisolierung (9) angeordnet ist.

14. Kühlauflage nach einem der Ansprüche 1 bis 13, **dadurch gekennzeichnet, dass** an der, dem Körper abgewandten Seite des zumindest einen Kühlelements (3) eine Reflexionsschicht (10) angeordnet ist.

15. Kühlauflage nach einem der Ansprüche 1 bis 14, **dadurch gekennzeichnet, dass** die Kühlelemente (3) aus Latex gebildet sind.

16. Kühlauflage nach einem der Ansprüche 1 bis 14, **dadurch gekennzeichnet, dass** die Kühlelemente (3) aus Silikon gebildet sind.

17. Kühlauflage nach einem der Ansprüche 1 bis 16, **dadurch gekennzeichnet, dass** an der, dem Körper zugewandten Seite des zumindest einen Kühlelements (3) eine Kontaktplatte (6) aus einem Material mit besonders hoher Wärmeleitfähigkeit angeordnet ist.

18. Kühlauflage nach einem der Ansprüche 7 bis 16, **dadurch gekennzeichnet, dass** die Unterlage (4) zumindest unterhalb der Kühlelemente (3) mit geringerer Dicke als die übrigen Bereiche ausgebildet ist.

19. Kühlauflage nach einem der Ansprüche 1 bis 18, **dadurch gekennzeichnet, dass** an dem zumindest einen Kühlelement (3) oder der Unterlage (4) Elemente (13) zur Verbindung mit anderen Kühlelementen (3) oder Unterlagen (4) vorgesehen sind.

20. Kühlauflage nach Anspruch 19, **dadurch gekennzeichnet, dass** die Verbindungselemente (13) durch Reißverschlüsse gebildet sind.

21. Kühlauflage nach einem der Ansprüche 1 bis 20, **dadurch gekennzeichnet, dass** an dem zumindest einen Kühlelement (3) oder der Unterlage (4) ein Element zur Fixierung am Patienten vorgesehen ist.

22. Kühlauflage nach Anspruch 21, **dadurch gekennzeichnet, dass** das Fixierelement durch einen Gurt vorzugsweise mit einem Schnellverschluss, beispielsweise einen Klettverschluss gebildet ist.

23. Kühlauflage nach einem der Ansprüche 1 bis 22, **dadurch gekennzeichnet, dass** an der dem Patienten (1) zugewandten Seite eine Klebeschicht angeordnet ist.

24. Kühlauflage nach einem der Ansprüche 1 bis 23, **dadurch gekennzeichnet, dass** zwischen den Kühlelementen (3) Einschnitte (22), Perforationen oder dgl. vorgesehen sind.

25. Kühlauflage nach einem der Ansprüche 1 bis 24, **dadurch gekennzeichnet, dass** Sensoren (12) zur Messung der Temperatur des Patienten (1) vorgesehen sind.

26. Kühlauflage nach einem der Ansprüche 1 bis 25, **dadurch gekennzeichnet, dass** eine elektrische Einrichtung zur Herzmassage angeordnet ist.

27. Kühlauflage nach einem der Ansprüche 1 bis 26, **dadurch gekennzeichnet, dass** die Kühlelemente (3) in Form einer Decke angeordnet sind.

28. Kühlauflage nach einem der Ansprüche 1 bis 26, **dadurch gekennzeichnet, dass** die Kühlelemente (3) in Form eines Schlafsackes angeordnet sind.

29. Kühlauflage nach einem der Ansprüche 1 bis 26, **dadurch gekennzeichnet, dass** die Kühlelemente (3) in Form einer Kopfbedeckung angeordnet sind.

30. Kühlauflage nach einem der Ansprüche 1 bis 26, **dadurch gekennzeichnet, dass** die Kühlelemente (3) in Form eines Schlauches zur Aufnahme der Arme oder Beine angeordnet sind.

31. Kühlauflage nach einem der Ansprüche 1 bis 26, **dadurch gekennzeichnet, dass** die Kühlelemente (3) in Form eines Fäustlings angeordnet sind.

32. Kühlauflage nach einem der Ansprüche 1 bis 26, **dadurch gekennzeichnet, dass** die Kühlelemente (3) in Form eines Strumpfes angeordnet sind.

33. Kühlauflage nach einem der Ansprüche 1 bis 32, **dadurch gekennzeichnet, dass** eine Codierung, vorzugsweise eine Farbcodierung angeordnet ist.

34. Einrichtung zum Kühlen zumindest von Teilen des Körpers von Patienten (1) mit zumindest einer Kühlauflage (2) nach einem der Ansprüche 1 bis 33, und einem Kühlgerät (14), **dadurch gekennzeichnet, dass** das Kühlgerät (14) zur Abkühlung der Kühlauflage (2) auf Temperaturen unter 0°C ausgebildet ist.

35. Kühleinrichtung nach Anspruch 34, **dadurch gekennzeichnet, dass** das Kühlgerät (14) durch ein elektrisch angetriebenes Kühlaggregat (15) gebildet ist.

36. Kühleinrichtung nach Anspruch 35, **dadurch gekennzeichnet, dass** das Kühlgerät (14) durch ein Peltier-Element gebildet ist.

37. Kühleinrichtung nach Anspruch 36, **dadurch gekennzeichnet, dass** das Kühlgerät (14) durch einen passiven Behälter (16) mit einer Wärmeisolierung (17) zur Aufnahme der Kühlauflage (2) gebildet ist.

38. Kühleinrichtung nach Anspruch 37, **dadurch gekennzeichnet, dass** die Wärmeisolierung (17) des Behälters (16) aus evakuierter Kieselsäure besteht.

39. Kühleinrichtung nach einem der Ansprüche 34 bis 38, **dadurch gekennzeichnet, dass** das Kühlgerät (14) in einer Patientenliege integriert ist.

40. Kühleinrichtung nach einem der Ansprüche 34 bis 39, **dadurch gekennzeichnet, dass** zumindest ein Sensor (18) zur Messung der Temperatur vorgesehen ist.

41. Kühleinrichtung nach Anspruch 40, **dadurch gekennzeichnet, dass** zumindest ein Temperatursensor (18) mit einer Auswertungseinheit (19) und allenfalls einer akustischen und bzw. oder visuellen Ausgabeeinheit (20) verbunden sind.

## Claims

1. A cover (2) for cooling at least a part of the body of a patient, including at least one cooling element (3) containing a cooling fluid (8) and intended for placement on the body or body part, which cooling element (3) is cooled to below the freezing point prior to its application, **characterized in that** a material (7) having a good thermal conductivity as compared to the cooling fluid (8) is contained in the cooling element (3) to absorb the cooling fluid (8).

2. A cooling cover according to claim 1, **characterized in that** the thermally well conductive material (7) is comprised of a metal wool made of a metal or metal alloy having a good thermal conductivity, e.g. aluminum, copper or steel.

3. A cooling cover according to claim 1, **characterized in that** the thermally well conductive material (7) is comprised of a metal foam made of a metal or metal alloy having a high thermal conductivity, e.g. aluminum, copper or steel.

4. A cooling cover according to claim 3, **characterized in that** the metal foam is an open-pore foam.

5. A cooling cover according to claim 1, **characterized in that** the thermally well conductive material is graphite.

6. A cooling cover according to any one of claims 1 to 5, **characterized in that** the cooling fluid (8) is comprised of water.

7. A cooling cover according to any one of claims 1 to 6, **characterized in that** several cooling elements (3) are arranged on a flexible support (4).

8. A cooling cover according to claim 7, **characterized in that** the support (4) is made of silicone.

9. A cooling cover according to claim 8, **characterized in that** the support (4) is made of latex.

10. A cooling cover according to any one of claims 1 to 9, **characterized in that** a heat-insulating layer (11) is provided for arrangement between the cooling elements (3), or the support (4), and the body or body part.

11. A cooling cover according to claim 9 or 10, **characterized in that** the support (4) includes a reinforcement layer.

12. A cooling cover according to any one of claims 1 to 11, **characterized in that** the at least one cooling element (3) is substantially designed with a parallelepipedal shape.

13. A cooling cover according to any one of claims 1 to 12, **characterized in that** a heat insulation (9) is arranged on the side facing away from the body, of the at least one cooling element (3).

14. A cooling cover according to any one of claims 1 to 13, **characterized in that** a reflection layer (10) is arranged on the side facing away from the body, of the at least one cooling element (3).

15. A cooling cover according to any one of claims 1 to 14, **characterized in that** the cooling elements (3) are made of latex.

16. A cooling cover according to any one of claims 1 to 14, **characterized in that** the cooling elements (3) are made of silicone.

17. A cooling cover according to any one of claims 1 to 16, **characterized in that** a contact plate (6) made of a material having a particularly high thermal conductivity is arranged on the side facing the body, of the at least one cooling element (3).

18. A cooling cover according to any one of claims 7 to 16, **characterized in that** the support (4) is designed to have a reduced thickness, at least below the cooling elements (3), as compared to the remaining regions.

19. A cooling cover according to any one of claims 1 to 18, **characterized in that** on the at least one cooling element (3) or on the support (4) means (13) for connection with other cooling elements (3) or supports (4), respectively, are provided.

20. A cooling cover according to claim 19, **characterized in that** the connection means (13) are formed by zippers.

21. A cooling cover according to any one of claims 1 to 20, **characterized in that** a means for fixing to the patient is provided on the at least one cooling element (3) or on the support (4).

22. A cooling cover according to claim 21, **characterized in that** the fixing means is comprised of a belt, preferably a belt with a quick-lock closure such as a Velcro closure.

23. A cooling cover according to any one of claims 1 to 22, **characterized in that** an adhesive layer is provided on the side facing the patient (1).

24. A cooling cover according to any one of claims 1 to 23, **characterized in that** incisions (22), perforations or the like are provided between the cooling elements (3).

25. A cooling cover according to any one of claims 1 to 24, **characterized in that** sensors (12) for measuring the temperature of the patient (1) are provided.

26. A cooling cover according to any one of claims 1 to 25, **characterized in that** an electric device for cardiac massage is provided.

27. A cooling cover according to any one of claims 1 to 26, **characterized in that** the cooling elements (3) are provided in the form of a blanket.

28. A cooling cover according to any one of claims 1 to 26, **characterized in that** the cooling elements (3) are provided in the form of a sleeping bag.

29. A cooling cover according to any one of claims 1 to 26, **characterized in that** the cooling elements (3) are provided in the form of a headgear.

30. A cooling cover according to any one of claims 1 to 26, **characterized in that** the cooling elements (3) are provided in the form of a flexible tube for receiving an arm or leg.

31. A cooling cover according to any one of claims 1 to 26, **characterized in that** the cooling elements (3) are provided in the form of a mitten.

32. A cooling cover according to any one of claims 1 to 26, **characterized in that** the cooling elements (3) are provided in the form of a stocking.

33. A cooling cover according to any one of claims 1 to 32, **characterized in that** a code, preferably a color code, is provided.

34. A device for cooling at least a part of the body of a patient (1), including at least one cooling cover (2) according to any one of claims 1 to 33 and a cooling appliance (14), **characterized in that** the cooling appliance (14) is designed to cool the cooling cover (2) to a temperature of below 0°C.

35. A cooling device according to claim 34, **characterized in that** the cooling appliance (14) is comprised of an electrically operated refrigerating unit (15).

36. A cooling device according to claim 35, **characterized in that** the cooling appliance (14) is comprised of a Peltier element.

37. A cooling device according to claim 36, **characterized in that** the cooling appliance (14) is comprised of a passive container (16) with a heat insulation (17) to receive the cooling cover (2).

38. A cooling device according to claim 37, **characterized in that** the heat insulation (17) of the container (16) is comprised of evacuated silicic acid.

39. A cooling device according to any one of claims 34 to 38, **characterized in that** the cooling appliance (14) is integrated in a patient gurney.

40. A cooling device according to any one of claims 34 to 39, **characterized in that** at least one sensor (18) for measuring the temperature is provided.

41. A cooling device according to claim 40, **characterized in that** at least one temperature sensor (18) is connected with an evaluation unit (19) and, optionally, an acoustic and/or visual output unit (20).

## Revendications

1. Système de compresse (2) pour refroidir au moins des parties du corps de patients (1), comprenant au moins un élément de refroidissement (3), qui renferme un liquide de refroidissement (8) et est destiné à être appliqué sur le corps ou la partie de corps, ledit élément de refroidissement (3) étant refroidi en-dessous du point de congélation avant l'utilisation, **caractérisé en ce qu'**à l'intérieur de l'élément de refroidissement (3) est logé un matériau (7) présentant une bonne conductibilité thermique par rapport au liquide de refroidissement (8), et destiné à recevoir le liquide de refroidissement (8).

2. Système de compresse de refroidissement ou compresse froide selon la revendication 1, **caractérisé en ce que** le matériau (7) de bonne conductibilité thermique est formé par de la laine métallique en un métal ou un alliage de métaux de bonne conductibilité thermique, par exemple de l'aluminium, du cuivre ou de l'acier.

3. Système de compresse de refroidissement selon la revendication 1, **caractérisé en ce que** le matériau (7) de bonne conductibilité thermique est formé par de la mousse métallique en un métal ou un alliage de métaux de bonne conductibilité thermique, par exemple de l'aluminium, du cuivre ou de l'acier.

4. Système de compresse de refroidissement selon la revendication 3, **caractérisé en ce que** la mousse métallique est à pores ouverts.

5. Système de compresse de refroidissement selon la revendication 1, **caractérisé en ce que** le matériau de bonne conductibilité thermique est formé par du graphite.

6. Système de compresse de refroidissement selon l'une des revendications 1 à 5, **caractérisé en ce que** le liquide de refroidissement (8) est formé par de l'eau.

7. Système de compresse de refroidissement selon l'une des revendications 1 à 6, **caractérisé en ce que** plusieurs éléments de refroidissement (3) sont agencés sur un support de base (4) flexible.

8. Système de compresse de refroidissement selon la revendication 7, **caractérisé en ce que** le support de base (4) est réalisé en silicone.

9. Système de compresse de refroidissement selon la revendication 7, **caractérisé en ce que** le support de base (4) est réalisé en latex.

10. Système de compresse de refroidissement selon l'une des revendications 1 à 9, **caractérisé en ce qu'**il est prévu une couche d'isolation thermique (11) destinée à être agencée entre les éléments de refroidissement (3) ou le support de base (4) et le corps ou les parties du corps.

11. Système de compresse de refroidissement selon la revendication 9 ou la revendication 10, **caractérisé en ce que** le support de base (4) renferme une couche de renfort.

12. Système de compresse de refroidissement selon l'une des revendications 1 à 11, **caractérisé en ce que** ledit au moins un élément de refroidissement (3) est d'une configuration sensiblement de forme parallélépipédique.

13. Système de compresse de refroidissement selon l'une des revendications 1 à 12, **caractérisé en ce qu'**une isolation thermique (9) est agencée sur le côté dudit au moins un élément de refroidissement (3), qui est à l'opposé de celui dirigé vers le corps.

14. Système de compresse de refroidissement selon l'une des revendications 1 à 13, **caractérisé en ce qu'**une couche de réflexion (10) est agencée sur le côté dudit au moins un élément de refroidissement (3), qui est à l'opposé de celui dirigé vers le corps.

15. Système de compresse de refroidissement selon l'une des revendications 1 à 14, **caractérisé en ce que** les éléments de refroidissement (3) sont réalisés en latex.

16. Système de compresse de refroidissement selon l'une des revendications 1 à 14, **caractérisé en ce que** les éléments de refroidissement (3) sont réalisés en silicone.

17. Système de compresse de refroidissement selon l'une des revendications 1 à 16, **caractérisé en ce que** sur le côté dudit au moins un élément de refroidissement (3), qui est dirigé vers le corps, est agencée une plaque de contact (6) en un matériau d'une conductibilité thermique particulièrement élevée.

18. Système de compresse de refroidissement selon l'une des revendications 7 à 16, **caractérisé en ce que** le support de base (4) est réalisé, au moins sous les éléments de refroidissement (3), avec une épaisseur plus faible que dans les autres zones.

19. Système de compresse de refroidissement selon l'une des revendications 1 à 18, **caractérisé en ce que** sur ledit au moins un élément de refroidissement (3) ou sur le support de base (4) sont prévus des éléments (13) pour assurer la liaison avec d'autres éléments de refroidissement (3) ou d'autres supports de base (4).

20. Système de compresse de refroidissement selon la revendication 19, **caractérisé en ce que** les éléments de liaison (13) sont formés par des fermetures à glissière ou fermetures Eclair.

21. Système de compresse de refroidissement selon l'une des revendications 1 à 20, **caractérisé en ce que** sur ledit au moins un élément de refroidissement (3) ou sur le support de base (4) est prévu un élément pour la fixation au patient.

22. Système de compresse de refroidissement selon la revendication 21, **caractérisé en ce que** l'élément de fixation est formé par une sangle comportant de préférence un système de fermeture rapide, par exemple une fermeture auto-agrippante du type Velcro.

23. Système de compresse de refroidissement selon l'une des revendications 1 à 22, **caractérisé en ce qu'**une couche adhésive est agencée sur le côté dirigé vers le patient (1).

24. Système de compresse de refroidissement selon l'une des revendications 1 à 23, **caractérisé en ce qu'**entre les éléments de refroidissement (3) sont prévus des incisions (22), des perforations ou analogues.

25. Système de compresse de refroidissement selon l'une des revendications 1 à 24, **caractérisé en ce que** sont prévus des capteurs (12) pour mesurer la température du patient (1).

26. Système de compresse de refroidissement selon l'une des revendications 1 à 25, **caractérisé en ce qu'**il y est agencé un dispositif de massage cardiaque.

27. Système de compresse de refroidissement selon l'une des revendications 1 à 26, **caractérisé en ce que** les éléments de refroidissement (3) sont agencés sous la forme d'une couverture.

28. Système de compresse de refroidissement selon l'une des revendications 1 à 26, **caractérisé en ce que** les éléments de refroidissement (3) sont agencés sous la forme d'un sac de couchage.

29. Système de compresse de refroidissement selon l'une des revendications 1 à 26, **caractérisé en ce que** les éléments de refroidissement (3) sont agencés sous la forme d'un bonnet ou d'une coiffe.

30. Système de compresse de refroidissement selon l'une des revendications 1 à 26, **caractérisé en ce que** les éléments de refroidissement (3) sont agencés sous la forme d'un boyau destiné à recevoir les bras ou les jambes.

31. Système de compresse de refroidissement selon l'une des revendications 1 à 26, **caractérisé en ce que** les éléments de refroidissement (3) sont agencés sous la forme d'une moufle.

32. Système de compresse de refroidissement selon l'une des revendications 1 à 26, **caractérisé en ce que** les éléments de refroidissement (3) sont agencés sous la forme d'un bas.

33. Système de compresse de refroidissement selon l'une des revendications 1 à 32, **caractérisé en ce qu'**il y est agencé un codage, de préférence un codage couleur.

34. Dispositif pour refroidir au moins des parties du corps de patients (1), à l'aide d'au moins un système de compresse de refroidissement (2) selon l'une des revendications 1 à 33, et d'un appareil de réfrigération (14), **caractérisé en ce que** l'appareil de réfrigération (14) est conçu pour refroidir le système de compresse de refroidissement (2) à des températures inférieures à 0°C.

35. Dispositif de refroidissement selon la revendication 34, **caractérisé en ce que** l'appareil de réfrigération (14) est formé par un groupe frigorifique (15) entraîné électriquement.

36. Dispositif de refroidissement selon la revendication 35, **caractérisé en ce que** l'appareil de réfrigération (14) est formé par un élément Peltier.

37. Dispositif de refroidissement selon la revendication 36, **caractérisé en ce que** l'appareil de réfrigération (14) est formé par un récipient ou conteneur passif (16) avec une isolation thermique (17), pour recevoir le système de compresse de refroidissement (2).

38. Dispositif de refroidissement selon la revendication 37, **caractérisé en ce que** l'isolation thermique (17) du récipient ou conteneur (16) est formé à base d'acide silicique sous vide.

39. Dispositif de refroidissement selon l'une des revendications 34 à 38, **caractérisé en ce que** l'appareil de réfrigération (14) est intégré dans un lit ou une couchette de patient.

40. Dispositif de refroidissement selon l'une des revendications 34 à 39, **caractérisé en ce qu'**il est prévu au moins un capteur (18) pour mesurer la température.

41. Dispositif de refroidissement selon la revendication 40, **caractérisé en ce qu'**au moins un capteur de température (18) est relié à une unité de traitement de données (19) et, dans tous les cas, à une unité de sortie (20) acoustique et, respectivement ou, visuelle.
